Europäisches Patentamt

European Patent Office

Office européen des brevets

(11) Veröffentlichungsnummer: **0 239 983 B1**

(19)

(12) # EUROPÄISCHE PATENTSCHRIFT

(45) Veröffentlichungstag der Patentschrift: **21.11.91**

(51) Int. Cl.⁵: **A61J 3/06**

(21) Anmeldenummer: **87104716.3**

(22) Anmeldetag: **31.03.87**

(54) Formlinge aus Pellets.

(30) Priorität: **01.04.86 DE 3610878**

(43) Veröffentlichungstag der Anmeldung:
**07.10.87 Patentblatt 87/41**

(45) Bekanntmachung des Hinweises auf die
Patenterteilung:
**21.11.91 Patentblatt 91/47**

(84) Benannte Vertragsstaaten:
**AT BE CH DE ES FR GB GR IT LI LU NL SE**

(56) Entgegenhaltungen:
DE-A- 1 767 320      DE-A- 2 128 527
DE-A- 2 336 218      DE-A- 3 532 692
FR-A- 2 011 960      FR-A- 2 343 473
US-A- 2 996 431

(73) Patentinhaber: **BOEHRINGER INGELHEIM KG**
**Postfach 200**
**W-6507 Ingelheim am Rhein(DE)**

(84) Benannte Vertragsstaaten:
**BE CH DE ES FR GR IT LI LU NL SE AT**

Patentinhaber: **BOEHRINGER INGELHEIM IN-**
**TERNATIONAL GmbH**
**Postfach 20**
**W-6507 Ingelheim am Rhein(DE)**

(84) Benannte Vertragsstaaten:
**GB**

(72) Erfinder: **Freund, Bernhard, Dr.**
**Karl-Domdey-Strasse 28**
**W-6535 Gau-Algesheim(DE)**
Erfinder: **Thies, Erwin**
**Platanen-Weg 21**
**W-6507 Ingelheim/Rhein(DE)**

## Beschreibung

Die Erfindung betrifft Formlinge aus Pellets.

Auf dem Gebiet der peroralen Depotformen nehmen retardierte wirkstoffhaltige Pellets eine bedeutende Stellung ein. Üblicherweise werden diese Pellets in Gelatinesteckkapseln abgefüllt, die vom Patienten ungeöffnet eingenommen werden. Ein großer Nachteil dieser Applikationsform ist, daß eine flexible Dosierung des retardierten Arzneimittels nur in einem ganzzahligen Vielfachen der Einzeldosis einer Steckkapsel möglich ist. Teilmengen des Arzneimittels können nicht unter kontrollierten Bedingungen, d.h. einer zuvor bestimmbaren und reproduzierbaren Dosis, vom Patienten entnommen werden. Die sichere Aufbewahrung der Restmenge an Arzneimittel ist nicht gewährleistet.

Zusätzlich werden viele Steckkapseln aus Gründen der Arzneimittelsicherheit mit einem Sicherheitsverschluß versehen, der beim Öffnen der Kapsel zu ihrer Zerstörung führt.

Viele Patienten empfinden es als ausgesprochen unangenehm, ein Arzneimittel in Form einer handelsüblichen Gelatinekapsel herunterzuschlukken, da die Kapsel als Fremdkörper empfunden wird. Diese Empfindung kann so ausgeprägt sein, daß der Schluckmechanismus völlig unterbunden wird.

In einem solchen Fall wäre es vorteilhaft, die Kapsel in wenig Wasser aufzulösen und die dabei freigesetzten Pellets als "wässerige Suspension" herunterzuschlucken. Wie jedoch bislang erst wenig bekannt ist, lösen sich die handelsüblichen Gelatinekapseln bei Raumtemperatur nicht in Wasser auf.

Es ist dem Fachmann bekannt, daß Pellets nicht zu Tabletten gepreßt werden können. Der hierbei notwendigerweise ausgeübte hohe Preßdruck führt Zumindest teilweise zu einer Zerstörung des Pelletkörpers, wodurch sich das Wirkstofffreigabeverhalten des Pellets nachteilig verändert. Vereinzelte Bemühungen, ein Pellet zu entwickeln, das dem beim Tablettenpressen notwendigen hohen Drucken standhält, sind mit drastischen Einschränkungen bei der Auswahl der Überzugsmaterialien und der Größe der Pellets verbunden (vgl. DE-A-23 36 218).

Diese Problematik regte dazu an, die Pellets in ein Bindemittel einzubetten, welches bei der Formgebung die Anwendung hoher Preßdrucke nicht mehr erforderlich machte. Die Herstellung eines derartigen Formkörpers ist in FR-A-2.011.960 beschrieben. Ein Nachteil des dort offenbarten Verfahrens besteht jedoch darin, daß die Pellets - und damit die darin enthaltenen Wirkstoffe - über den größten Teil des Herstellungsprozesses einer erhöhten Temperatur ausgesetzt sind, was für thermolabile Wirkstoffe mit großen Risiken verbunden ist. Ferner zerfällt der nach dem in dieser Offenlegungsschrift offenbarten Verfahren hergestellte kompakte Formkörper im wässerigen Milieu nur sehr langsam.

Es ist somit die Aufgabe der vorliegenden Erfindung, eine galenische Form für Pellets vorzuschlagen, die in einer annehmbaren Zeitspanne in einem wässerigen Milieu zerfällt und die Pellets freigibt.

Die Aufgabe der vorliegenden Erfindung wird durch einen Formling gelöst, bei dem Pellets in einer schmelzbaren (sinterbaren) Matrix eingebettet sind, wobei der Formling gegebenenfalls Vorrichtungen, wie z.B. Kerben, für eine bessere Teilung enthält.

Ein zusätzlicher Vorteil des erfindungsgemäßen Verfahrens besteht darin, daß im Verlauf des Herstellungsverfahrens nicht die beim Tablettenpressen üblicherweise erforderlich hohen Pressdrucke aufgewendet werden müssen.

Ein weiterer Vorteil besteht darin, daß durch das erfindungsgemäße Verfahren die Bereitstellung einer ein-oder mehrfach teilbaren galenischen Form für Pellets ermöglicht wird, die eine Teilung unter Erhalt der für Pellets charakteristischen Eigenschaften ermöglicht.

Der Begriff schmelzbare oder sinterbare Matrix bezieht sich auf eine Matrix, die im Verlauf ihres Herstellungsverfahrens entweder durch Erstarren einer Schmelze oder durch Sintern (kurzzeitiges Erhitzen) eines pulverförmigen Materials verfestigt wird.

Unter dem Begriff Formling sind erfindungsgemäß nicht nur tabletten- oder kapselähnliche Formen, sondern alle Ausführungen zu verstehen, die eine Form bilden und für eine orale Applikation geeignet sind.

Bei dem erfindungsgemäßen Formling werden keine einschränkenden Anforderungen an die Stabilität der Pelletumhüllung wie auch an die Größe der Pellets gestellt. Als Pellets sind alle nach bekannten Dragier-bzw. Überzugs-Verfahren hergestellten Pellets geeignet. Hierzu zählen unretardierte (d.h. direkt lösliche), magensaftresistent überzogene sowie retardierte Pellets. Die Matrix besteht aus Bindemitteln und gegebenenfalls Zusatzstoffen, wie sie üblicherweise in der Galenik Verwendung finden. Das Bindemittel sollte wasserlöslich oder zumindest in Wasser emulgierbar sein, gegebenenfalls in Abhängigkeit des pH-Wertes, und vorzugsweise einen Schmelzpunkt unter 100° C aufweisen.

Bevorzugte Bindemittel sind Zuckeralkohole, wie beispielsweise Mannit, Xylit oder Sorbit, sowie deren Ester Genußsäuren, wie beispielsweise Weinsäure, Zitronensäure, Äpfelsäure, Lactid oder Milchsäure; Fettsäuren, sowie deren Mono- oder Diglyceride, Zuckermischungen, wie z.B. Mono- oder Disaccharide oder Glukosesirup, Mono- oder Diglyceride und deren Derivate oder Gemische der genannten Stoffe.

Bei Hilfsstoffen mit hohen Schmelzpunkten finden bevorzugt eutektische Gemische mit einem Schmelzpunkt unterhalb 100° C Verwendung.

Ein geeignetes Bindemittel für eine schmelzbare Matrix ist ein Gemisch aus Zucker, Milchzucker, Sorbit, Stärkesirup und Wasser, das in der Literatur auch als "Basic Mix" beschrieben wird. "Basic Mix" ist eine glasartige Masse mit einem Erweichungspunkt von 55 bis 90° C und einer Restwassermenge von ca. 0,5 bis 2 %.

Besonders bevorzugte Bindemittel sind Polyethylenglykole, wie beispielsweise Polywachs 1500, Polywachs 6000 oder Polywachs 20 000, die einen Erstarrungspunkt zwischen 25° und 60° C aufweisen.

Erfindungsgemäße Formlinge, die als Bindemittel Polyethylenglykole enthalten, stellen eine bevorzugte Ausführungsform dar, da der Formling innerhalb einer kurzen Zeitspanne in einem wässerigen Milieu schnell zerfällt und die Pellets als eine Art "wässriger Suspension" bequem geschluckt werden können.

Ein weiteres Kriterium für die Auswahl des Bindemittels ist, daß es mit der retardierten Polymerhülle der Pellets kompatibel ist.

Zur Herstellung der erfindungsgemäßen Formlinge eignen sich die nachfolgend beschriebenen Herstellungsverfahren:

a) Pellets, die nach literaturbekannten Verfahren hergestellt und mit einem retardierenden Überzug versehen sind, werden in geeignete Formen gefüllt, und die Form anschließend mit einer ausreichenden Menge des geschmolzenen Bindemittels ausgegossen. Die zu verwendende Menge an Bindemittel, gegebenenfalls mit galenischen Zusatzstoffen versetzt, beträgt zwischen 5 und 50 Gew.-%, bevorzugt 10 - 25 Gew.-%, bezogen auf die Menge der Pellets. Nach dem Erkalten werden die fertigen Formlinge aus der Form herausgenommen und konfektioniert. Gegebenenfalls kann zur besseren Entnahme zusätzlich ein Trennmittel zuvor auf die Form aufgebracht werden. Geeignete Formen sind solche, die eine einfache Entnahme des erkalteten Formlings ermöglichen.

b) In einem weiteren erfindungsgemäßen Verfahren wird auf die bereits retardierten Pellets das Bindemittel in geeigneter Dicke aufgetragen. Dies kann beispielsweise im Dragierverfahren erfolgen. Die Dicke der Bindemittelschicht hängt im wesentlichen von der Größe der Pellets ab und kann durch einfache Versuche ermittelt werden. Die Menge an verwendetem Bindemittel liegt im allgemeinen zwischen 5 und 50 Gew.-%, bevorzugt zwischen 10 und 25 Gew.-%, bezogen auf die Menge der Pellets.

Die mit dem Bindemittel überzogenen Pellets werden in Formen gefüllt und durch kurzes Erhitzen, wobei das Bindemittel zumindest auf der Oberfläche kurzzeitig schmilzt, miteinander verbunden. Die zum Schmelzen des Bindemittels erforderliche Wärme kann bei entsprechend ausgestalteten Formen über die Form zugeführt werden, die gegebenenfalls zusätzlich auch über Abkühlvorrichtungen verfügt. Ist eine Temperaturzuführung über die Form nicht möglich, kann die zum Aufschmelzen benötigte Energie "von außen", beispielsweise durch Mikrowellenstrahlung oder einer Heizeinrichtung, wie sie beispielsweise ein einfacher Ofen darstellt, zugeführt werden. Es ist lediglich sicherzustellen, daß die Retardschicht der Pellets nicht durch ein übermäßiges Erwärmen geschädigt wird.

c) In einem weiteren erfindungsgemäßen Verfahren werden die retardierten Pellets zusammen mit dem Bindemittel gemischt und in eine Form gefüllt. Die weitere Verarbeitung erfolgt wie unter b) beschrieben.

d) Bei einem weiteren erfindungsgemäßen Herstellungsverfahren werden die in üblicherweise retardierten Pellets zusammen mit dem Bindemittel in eine Form gegeben. Beim Erhitzen wird das Bindemittel durch die Zersetzung eines Treibmittels gleichmäßig zwischen den Pellets verteilt, so daß nach dem Abkühlen der gewünschte Formling entstanden ist. Als Treibmittel sind solche Verbindungen geeignet, die pharmakologisch unbedenklich sind und einen Zersetzungspunkt im Bereich des Schmelzpunktes des Bindemittels aufweisen. Weiterhin sind niedrig siedende Lösungsmittel, wie z.B. Kohlenwasserstoffe, halogenierte Kohlenwasserstoffe, perhalogenierte Kohlenwasserstoffe, Alkohole, als Treibmittel geeignet, sofern diese mit dem retardierenden Überzug verträglich sind. Bei speziell gestalteten Formen kann als Treibmittel auch ein inertes Gas, z.B. Preßluft, Stickstoff, Verwendung finden.

Ein weiteres Verfahren zur Herstellung der erfindungsgemäßen Formlinge besteht darin, daß die Pellets zusammen mit einem Bindemittel gemischt und anschließend extrudiert werden. Hierbei kann der Extruder derart gestaltet werden, daß der extrudierte Strang, bestehend aus den Pellets und dem Bindemittel, in vorgegebenen Intervallen Einkerbungen zur Teilung in einzelne Formlinge erhält. In einer besonderen Ausführungsform ist der extrudierte Strang so lang wie der Formling.

Die Mischung des Bindemittels und der Pellets ist selbstverständlich so durchzuführen, daß die retardierte Hülle nicht beschädigt wird. Entsprechende Vorkehrungen sind auch für die Extrusion zu treffen. Vorteilhafterweise sollte das Bindemittel einen breiten Erweichungspunkt aufweisen.

Beim Erhitzen (Sintern) des Formkörpers, insbesondere bei den beschriebenen Verfahren b) und c), erfolgt durch die Erweichung der Pelletschüttung eine geringfügige Volumenkontraktion. Zur Vermeidung von Rissen und zur Erzielung eines einheitlichen Aussehens ist es von Vorteil, auf den noch warmen Formling einen geringen Druck auszuüben, um die gewünschte Formgebung zu unterstützen. Hierbei ist es jedoch selbstverständlich, daß die angewandten Drucke so gering sind, daß die Pellets, insbesondere die Retardierung der Pellets, nicht beschädigt werden können. Bei einer geeigneten Ausgestaltung der Formen ist es ausreichend, wenn die Form kurzzeitig gerüttelt wird, um eine Verdichtung zu erreichen.

In einer besonderen Ausführungsform des erfindungsgemäßen Herstellungsverfahrens werden tiefgezogene Folien aus Kunststoff mit entsprechenden Ausformungen für den Formling (mit gegebenenfalls zusätzlichen Ausformungen zur Ausbildung der Teilkerben) verwendet.

Die Füllung der Folien und die Herstellung der Formlinge erfolgt nach einem der unter a - d beschriebenen erfindungsgemäßen Verfahren, wobei die Folie gleichzeitig als Form verwendet wird. Nach dem Erkalten der Formlinge wird die Folie mit Aluminiumfolie versiegelt. Zum Gebrauch werden die Formlinge durch die Aluminiumfolie herausgedrückt.

Ein nach dem Verfahren b, c oder d hergestellter Formling kann je nach Menge des verwendeten Bindemittels eine unebene Oberfläche aufweisen, dies hat jedoch keinen Einfluß auf das Wirkstofffreigabeverhalten.

Nach dem Verfahren b hergestellte Formlinge weisen eine poröse innere Struktur auf. Die Verbindung zwischen den Pellets besteht nur an den jeweiligen Berührungspunkten. Derartige Formlinge besitzen daher günstigere Zerfallseigenschaften, da Wasser oder die entsprechenden Verdauungsflüssigkeiten schneller eindringen und das Bindemittel auflösen können.

Wenn es gewünscht wird, kann durch einen weiteren Auftrag von Bindemittel oder einer Lackierung auf den Formling eine angenähert ebene Oberfläche erhalten werden.

In einer besonderen Ausführungsform können die erfindungsgemäßen Formlinge zusätzlich mit einer magensaftresistenten oder retardierenden Lackierung überzogen werden

In einer besonderen Ausführungsform können auch magensaftresistente Bindemittel eingesetzt werden, so daß die Pelletfreigabe erst in der Darmpassage erfolgt.

Vorrichtungen zur Teilung der Formlinge, z.B.

in Kerbenform, können beispielsweise durch entsprechend ausgestaltete Formen, oder aber auch nachträglich mit einem erwärmten Werkzeug oder aber in den noch weichen, d.h. nicht abgekühlten Formling, durch eine Bearbeitung mit einem entsprechend ausgestalteten Werkzeug eingebracht werden.

Ein wesentlicher Vorteil des erfindungsgemäßen Formlings aus Pellets ist, daß bei der erfindungsgemäßen Herstellung keine mechanische Beschädigung der Pellets erfolgt, hierdurch bleiben die retardierenden Eigenschaften der Pellets, insbesondere auch nach einer Teilung des Formlings, voll erhalten. Es ist selbstverständlich, daß die erfindungsgemäßen Formlinge aus Pellets auch aus solchen Pellets hergestellt werden können, die nicht mit einer retardierenden oder magensaftresistenten Umhüllung versehen sind.

Die im folgenden aufgeführten Ausführungsbeispiele sollen die Erfindung verdeutlichen, ohne sie jedoch auf die dargestellten Formen zu beschränken:

Die in Beispiel 1 dargestellten Formen wurden unter Verwendung nach herkömmlichen Verfahren retardierten Pellets hergestellt.

Beispiel 1

| | |
|---|---|
| Zu Abb. 1 | Pellets dieser Charge werden in die Näpfe für Kapseln in Tiefziehfolie eingefüllt und mit Polywachs 6000 ausgegossen. |
| Zu Abb. 2 | Pellets gleicher Charge werden im Mengenverhältnis 1 : 1 an pulverförmigen Polywachs 6000 in eine kapselähnliche Form gefüllt, bei ca. 65° C geschmolzen und ineinandergeschoben. Die Form wird nach dem Erkalten entfernt. |
| Zu Abb. 3 | Darstellung einer möglichen Teilkerbe. |
| Zu Abb. 4 | Pellets der gleichen Charge werden mit 20% Polywachs 20 000 im Becherglas erwärmt und anschließend bis zum völligen Erstarren mit dem Glastab gerührt. Die so mit Polywachs überzogenen Pellets werden in eine kapselähnliche Form mit Ober- und Unterteil gefüllt und auf ca. 65° C erhitzt, wobei die Form dabei langsam zusammengeschoben.wird. Nach dem Erkalten wird die Form entfernt. |
| Zu Abb. 5 | In ein Formunterteil werden 15 - 20% Polywachs 20000 und wenig Ammoniumbicarbonat gegeben und darauf die Pellets eingefüllt. |

Anschließend wird ein Formoberteil mit mehreren Löchern aufgeschoben und bei ca. 65° C gesintert. Der Überschuß an Gas und Polywachs kann entweichen.

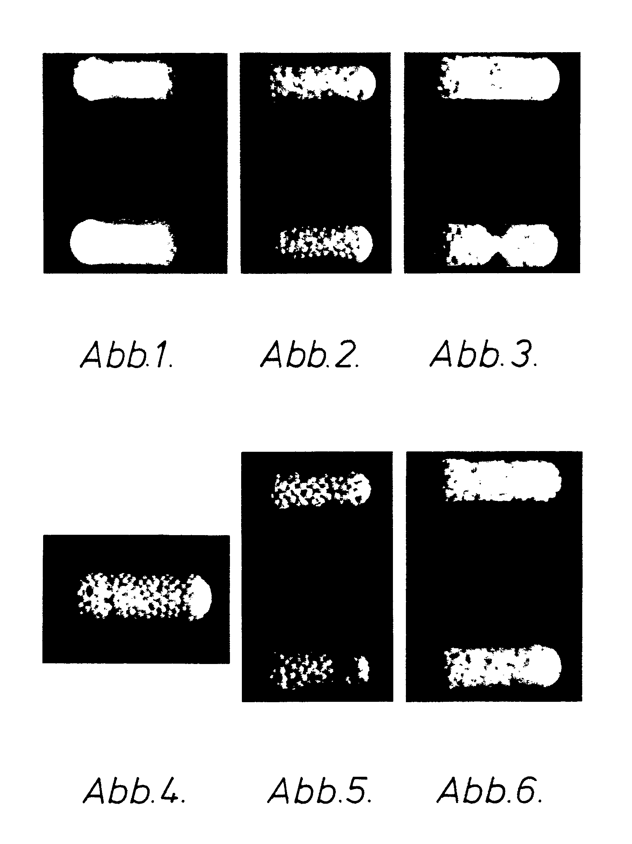

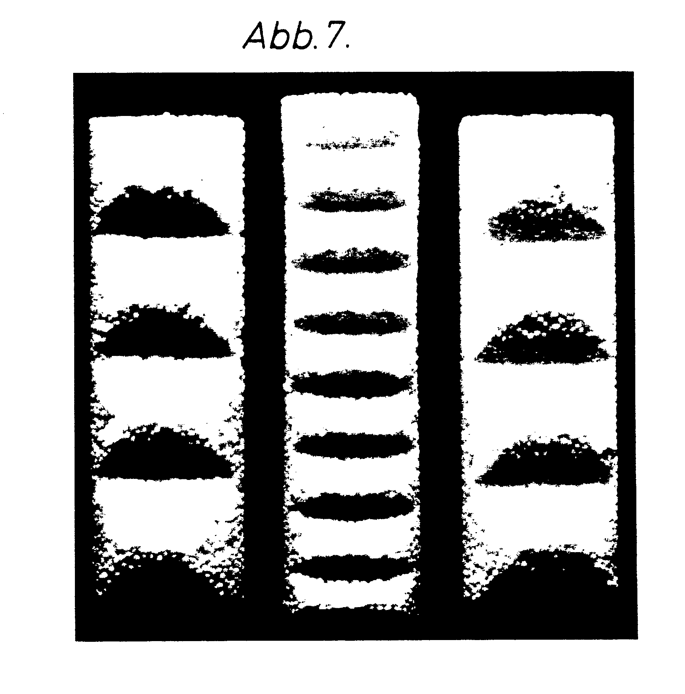

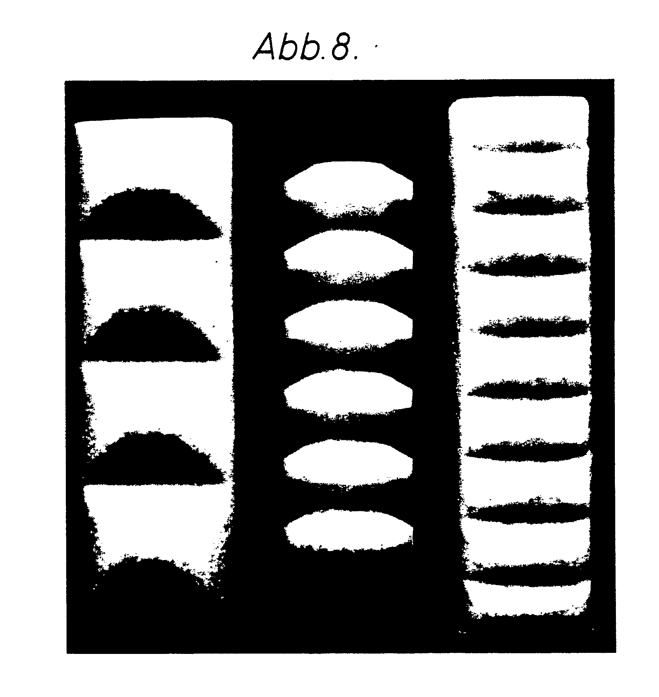

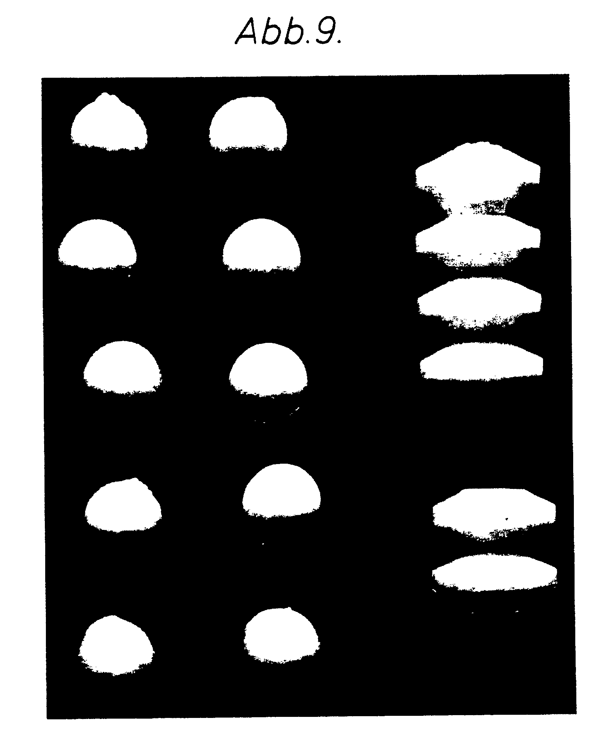

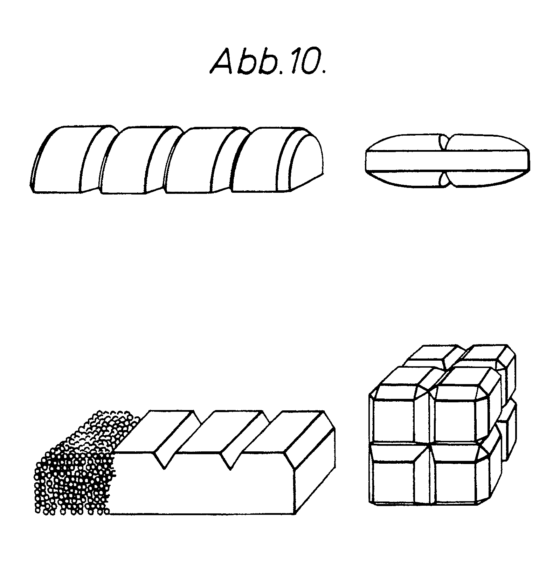

Zu Abb. 6 Die Formlinge werden auf die gleiche Weise hergestellt wie unter 4. Der obere Formling enthält 20 %, der untere 30 % Polywachs 20000.

Die Abbildungen zeigen vergrößerte Formlinge.

Zusätzlich zu den zuvor beschriebenen Beispielen werden Stangenformen hergestellt um den Gestaltungsreichtum der erfindungsgemäßen teilbaren Formlinge zu unterstreichen.

Beispiel 2

Placebopellets der Siebfraktion 0,6 - 0,71 mm werden im Dragierkessel mit einem Überzug von 20 Gewichtsprozent Polyethylenglykol 1500 versehen.

Die Formen werden durch Gießen aus Siliconkautschuk hergestellt unter Zuhilfenahme von feinmechanisch hergestelten Stangenmodellen aus Aluminium.

Bei direktem Sintern obiger Pellets im Mikrowellenherd und leichtem Eindrücken der Form von Hand entstehen die in Abb. 7 dargestellten Stangen.

Zur Schließung der Zwischenräume werden auch Formlinge hergestellt, die aus Pellets sowie einer zusätzlichen "äußeren Phase" an pulverförmige Polyethylenglykol bestehen (Abb. 8).

Die beschriebenen plastisch-elastischen Formen erlauben auch die Herstellung hinterschnittener Formlinge (Abb. 9).

In Abb. 10 sind einige mögliche Ausführungsformen der erfindungsgemäßen Formlinge dargestellt.

**Patentansprüche**

1. Verfahren zur Herstellung eines Formlings aus Pellets mit verzögerter kontrollierter Wirkstofffreigabe in einer schmelzbaren Matrix aus einem pharmakologisch verträglichen Bindemittel, dadurch gekennzeichnet, daß man entweder

   a) die Pellets in Formen füllt und diese anschließend mit dem geschmolzenen Bindemittel ausgießt,
   oder
   b) die Pellets mit dem Bindemittel umhüllt, in Formen füllt und anschließend kurzzeitig erhitzt,
   oder

   c) die Pellets mit dem Bindemittel mischt, in Formen füllt und anschließend kurzzeitig erhitzt,
   oder
   d) die Pellets zusammen mit dem Bindemittel und einem Treibmittel in Formen füllt und kurzzeitig erhitzt,

   sodaß ein in wässerigen Medien schnell zerfallender Formling mit einer porösen Struktur entsteht.

2. Verfahren nach Anspruch 1, dadurch gekennzeichnet, daß auf den noch warmen Formkörper zur Vermeidung von Rissen und zur Erzielung eines einheitlichen Aussehens ein geringer Druck zur Unterstützung der Formgebung ausgeübt wird.

3. Verfahren nach Anspruch 1 oder 2, dadurch gekennzeichnet, daß man als Form eine tiefgezogene Form verwendet.

4. Verfahren nach Anspruch 1, dadurch gekennzeichnet, daß die Pellets mit einem Bindemittel gemischt und anschließend zu einem Strang extrudiert werden, der gegebenenfalls Vorrichtungen zur Teilung enthält.

5. Formkörper, bestehend aus Pellets mit verzögerter Wirkstofffreigabe in einer schmelzbaren Matrix aus einem pharmakologisch verträglichen Bindemittel, dadurch gekennzeichnet, daß er nach einem der in Anspruch 1 beschriebenen Verfahren a bis d hergestellt worden ist.

**Claims**

1. A process for the manufacture of a moulding from pellets having a retarded controlled release of active substance, in a fusible matrix of a pharmacologically acceptable binder, characterised in that either

   a) the pellets are introduced into moulds and these are subsequently filled by pouring in a fused binder or
   b) the pellets are coated with a binder, introduced into moulds and then heated briefly or
   c) the pellets are mixed with a binder, introduced into moulds and then heated briefly or
   d) the pellets together with a binder and a blowing agent are introduced into moulds and heated briefly, so as to obtain a having a porous structure which decomposes rapidly in aqueous media.

2. A process according to claim 1, characterised in that in order to avoid cracks and achieve a uniform appearance, a slight pressure is exerted on the moulding, while it is still warm, to assist the moulding action.

3. A process according to claim 1 or 2, characterised in that a thermal formed mould (blister foil well) is used as the mould.

4. A process according to claim 1, characterised in that the pellets are mixed with a binder and then extruded to form a strip which optionally contains means for dividing it up.

5. A moulding consisting of pellets with a delayed release of active substance in a fusible matrix of a pharmacologically acceptable binder, characterised in that it has been prepared by one of processes a to d described in claim 1.

**Revendications**

1. Procédé de préparation d'un aggloméré à partir de pellets à libération contrôlée et retardée de la substance active dans une matrice fusible constituée par un liant compatible du point de vue pharmacologique, caractérisé en ce que soit

a) on introduit les pellets dans des moules et on les réunit ensuite avec le liant fondu, ou

b) on entoure les pellets par le liant, on les introduit dans des moules et on chauffe ensuite brièvement, ou

c) on mélange les pellets avec le liant, on les introduit dans des moules puis on chauffe brièvement, ou

d) on introduit les pellets dans des moules en même temps que le liant et un véhicule et on chauffe brièvement, de sorte qu'il en résulte un aggloméré à structure poreuse qui se désagrège rapidement dans les milieux aqueux.

2. Procédé selon la revendication 1, caractérisé en ce que, pour favoriser le façonnage, une faible pression est exercée sur l'aggloméré encore chaud pour éviter des fissures et pour obtenir un aspect uniforme.

3. Procédé selon la revendication 1 ou 2, caractérisé en ce que l'on utilise comme moule un moule ayant subi un emboutissage profond.

4. Procédé selon la revendication 1, caractérisé en ce que les pellets sont mélangés à un liant puis sont extrudés en une barre qui contient éventuellement des dispositifs pour le fractionnement.

5. Aggloméré consistant en pellets à libération retardée de la substance active dans une matrice fusible en un liant compatible du point de vue pharmacologique, caractérisé en ce qu'il a été préparé selon l'un des procédés a à d décrits dans la revendication 1.

Abb.1.     Abb.2.     Abb.3.

Abb.4.     Abb.5.     Abb.6.

## Abb.7.

## Abb.8.

Abb.9.

# Abb.10.